(19) Europäisches Patentamt
European Patent Office
Office européen des brevets

(11) Numéro de publication: **0 283 329**
**A1**

(12) # DEMANDE DE BREVET EUROPEEN

(21) Numéro de dépôt: **88400220.5**

(22) Date de dépôt: **01.02.88**

(51) Int. Cl.⁴: **C 07 D 519/04**
A 61 K 39/395, A 61 K 47/00

(30) Priorité: **03.02.87 FR 8701257**

(43) Date de publication de la demande:
**21.09.88 Bulletin 88/38**

(84) Etats contractants désignés:
**AT BE CH DE ES FR GB GR IT LI LU NL SE**

(71) Demandeur: **IRE-CELLTARG S.A.**
**Zone Industrielle**
**B-6220 Fleurus (BE)**

(72) Inventeur: **Bhushana Rao, Siva**
**11, rue Kwakenbienne**
**B-1331 Rosieres (BE)**

**Collard, Marie-Paule**
**18, rue Evenepoel**
**B-1040 Bruxelles (BE)**

**Trouet, André**
**29, Predikherenberg**
**B-3009 Winksele (BE)**

(74) Mandataire: **Warcoin, Jacques et al**
**Cabinet Régimbeau 26, avenue Kléber**
**F-75116 Paris (FR)**

(54) **Conjugués d'hydrazide d'alcaloide de vinca lie a une immunoglobuline.**

(57) L'invention concerne des conjugués actifs incorporant une substance à activité anticancéreuse et un agent de pilotage du type immunoglobuline, la substance à activité cancéreuse et l'agent de pilotage étant liés par un lien covalent du type hydrazide. Ils sont constitués de dérivés d'hydrazide d'alcaloïde de vinca lié à une immunoglobuline.

L'invention concerne également un procédé de préparation de ces conjugués actifs et des compositions pharmaceutiques les comprenant.

EP 0 283 329 A1

**Description**

"CONJUGUES D'HYDRAZIDE D'ALCALOÏDE DE VINCA LIE A UNE IMMUNOGLOBULINE".

Le domaine technique de la présente invention est généralement celui des conjugués actifs comportant un agent de pilotage, par example tel que défini dans la demande de brevet n° 85 10234, lié par un lien covalent à une substance à activité pharmacologique ou à accumulation intracellulaire dans des cellules cibles.

Plus précisément, la présente invention concerne des conjugués actifs incorporant des médicaments anticancéreux, choisis dans la famille des dérivés d'alcaloïdes de vinca, par exemple parmi la vinblastine ou la vindésine.

La vinblastine est un alcaloïde antitumoral connu, isolé à partir de Vinca rosea. La vinblastine (ci-après dénommée VBL) a été l'objet de nombreuses recherches, études et brevets en vue de la préparation de substances antitumorales plus actives, plus sélectives et moins toxiques.

La présente invention vise à obtenir des composés encore plus spécifiques de la maladie ou de la tumeur à traiter. En effet, les compositions comportant par example de la vinblastine, doivent être administrées actuellement, à de fortes concentrations ou principe actif, en vue d'atteindre l'efficacité désirée. Ces fortes concentrations entraînent inévitablement des effets secondaires.

Des liens covalents sont déjà connus de la technique antérieure et il faut citer, par example, les bras hémisuccinyl déjà utilisés pour coupler la vindésine en position $C_4$ et le bras tétrapeptidique déjà utilisé pour coupler la daunorubicine, la doxorubicine et la primaquine.

La présente invention propose un lien du type hydrazide, c'est-à-dire qu'un dérivé de la substance à activité pharmacologique du type $R-CO-NH-NH_2$ sera conjugué à un anticorps monoclonal, agent de pilotage.

Dans l'Art Antérieur, ce type de conjugués incorporant des dérivés hydrazides de vinblastine n'avaient jamais pu être obtenus.

La présente invention concerne donc particulièrement les hydrazides de vinblastine, composés de formule générale I :

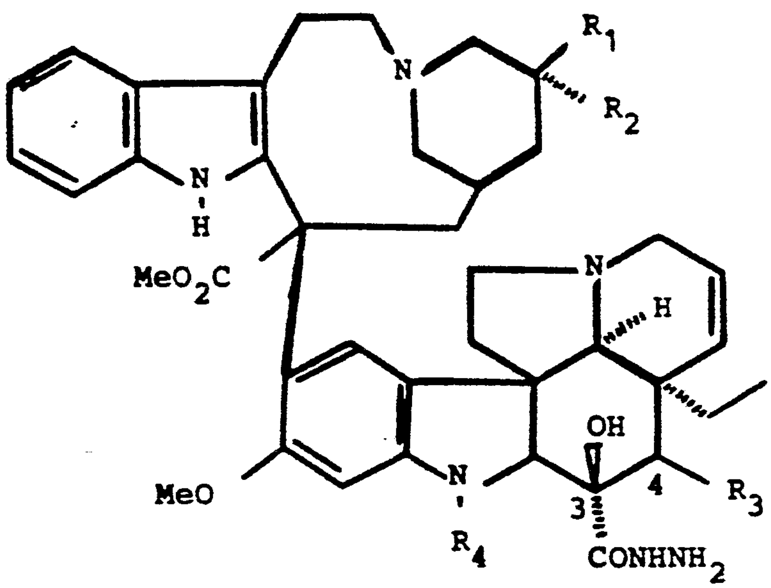

dans laquelle :

. ($R_1$, $R_2$) représentent (-Et, -OH) ou (-H, Et)
. $R_4$ représente -H, -Me ou -CHO,
. $R_3$ représente -OH ou $-O-\underset{\underset{O}{\|}}{C}-R'_3$.
. $R'_3$ représente un radical hydrocarboné ayant de 1 à 20 atomes de carbone, le radical hydrocarboné pouvant être non substitué ou substitué par un ou plusieurs radicaux amino, di ($C_1$-$C_3$ alkyl)amino, mono ($C_1$-$C_3$ alkyl)amino, NH ($C_2$-$C_5$)alcanoyl, cyano, COOH, S-($C_1$-$C_3$ alkyl), COO-($C_1$-$C_3$) alkyl ou COO-aryle.

Dans le cadre de la présente invention, mais de manière non limitative, on utilisera plus particulièrement à titre d'Ac comme agent de pilotage un anti-corps monoclonal d'origine animale ou humaine, par exemple une immunoglobuline du type IgG. De façon encore plus précise, on utilisera un Ac monoclonal dressé contre la membrane de globules graisseux de lait humain ou contre des antigènes de la membrane plasmique de cellules de carcinomes humains..

La présente invention a donc particulièrement pour objet des conjugués originaux, caractérisés en ce qu'ils

sont constitués d'hydrazides de vinblastine liés à une immunoglobuline.

Ces composés permettent en effet de réduire considérablement les doses à administrer tout en atteignant l'efficacité désirée.

Selon une caractéristique particulière de l'invention, la liaison entre l'hydrazide et l'immunoglobuline est réalisée au niveau de la région Fc de celle-ci.

Une immunoglobuline est un anticorps qui, très schématiquement, a une structure en forme de Y. Les "branches" de Y forment la partie reconnaissante du tissu cible et éventuellement de l'antigène caractéristique de la maladie.

Si l'on fixe des molécules douées d'une activité thérapeutique sur cette partie, la reconnaissance du tissu cible sera gênée. C'est pourquoi, selon la présente invention, on fixe l'hydrazide de vinblastine sur la partie non-reconnaissante ou région Fc de l'immunoglobuline. De cette façon, le tissu à soigner est mieux ciblé, et par conséquent, une dose relativement faible de principe actif est nécessaire pour combattre la maladie.

L'invention a également pour objet un procédé de préparation de ces dérivés conjugués.

Selon la caractéristique essentielle du procédé, on fait réagir dans un solvant organique l'hydrazide de vinblastine sur une immunoglobuline oxydée en aldéhyde dans sa partie sucre.

Avantageusement, la réaction de l'hydrazide de vinblastine a lieu sur la région Fc de l'immunoglobuline oxydée.

D'autres caractéristiques et avantages de la présente invention apparaîtront à la lecture des exemples qui suivent.

Les immunoglobulines ont été oxydées et conjuguées selon deux méthodes.

Exemple 1 : Oxydation d'immunoglobulines G non-spécifiques de chèvres

Procédure 1a

Une solution de 2 mg/ml d'immunoglobuline G dans un tampon de phosphate de sodium (pH = 6 ; 0,05 M) est placée au froid. A cette solution, on ajoute un dixième en volume d'une solution à 100 mM de $NaIO_4$. Le mélange est gardé au froid et à l'obscurité pendant une heure, puis on le passe à travers une colonne Sephadex G25 (1,6 x 38 cm) équilibrée avec un tampon de phosphate de sodium (pH 6, 0,05 M). Le pic d'exclusion est collecté et concentré à 2 mg/ml.

Procédure 1b

A une solution de 4 mg/ml d'immunoglobuline G dans l'eau, on ajoute 0,5 ml d'une solution de $NaIO_4$ (0,15 M). Le mélange est agité pendant 30 minutes à la température ambiante,puis dialysé contre de l'acétate de sodium (0,001 M, pH 4,4). Le même tampon est renouvelé trois fois.

Le mécanisme réactionnel est une oxydation de la partie hydrocarbonée de l'immunoglobuline en aldéhyde.

Exemple 2 : conjugaison d'immunoglobulines G non-spécifiques de chèvre

Procédure 2a

Une solution stable à 5mM d'hydrazide de vinblastine ($VBL\text{-}NH\text{-}NH_2$) marquée, est préparée en dissolvant 3,81 mg de $VBL\text{-}NH\text{-}NH_2$ dans 50 µl de dioxanne, et 950 µl d'une solution de phosphate de sodium (pH = 7 ; 0,01 M). A l'immunoglobuline G oxydée (procédure 1a : 2 mg/ml, phosphate de sodium pH 7, 0,01 M), on ajoute le $VBL\text{-}NH\text{-}NH_2$ jusqu'à une concentration de 500 µM. Le mélange est agité pendant 4 heures à la température ambiante, conservé au froid pendant une nuit. Le conjugué est ensuite isolé par filtration sur gel à l'aide d'une colonne Sephadex G 25 (2,6 x 90 cm) équilibrée avec du phosphate de sodium (0,05 M ; pH 6). On récupère le pic d'exclusion, on le concentre, on effectue le dosage en protéines par la méthode de Lowry et en $VBL\text{-}NH\text{-}NH_2$ par radioactivité. Le conjugué ainsi préparé contient 1,7 moles de $VBL\text{-}NH\text{-}NH_2$ par mole d'immunoglobuline G. Par filtration sur gel, on détecte 95 % de monomère d'IgG.

Procédure 2b

A 25 mg d'IgG oxydée (procédure 1b) ont été ajoutés 2 ml de solution stable 5 mM de $VBL\text{-}NH\text{-}NH_2$ marquée, ainsi que 2 ml de $NaHCO_3$ (0,5 M, pH : 9,5). Le mélange a été agité à 4°C pendant une nuit et le $VBL\text{-}NH\text{-}NH_2$ inaltéré a été éliminé par dialyse contre du NaCL 9 % pendant quatre jours à 4°C. Le conjugué a été concentré et dosé en protéine par la méthode Lowry et en $VBL\text{-}NH\text{-}NH_2$ par radioactivité. Le conjugué ainsi préparé contient deux moles de $VBL\text{-}NH\text{-}NH_2$ par mole d'IgG. Par filtration sur gel HPLC, il a été détecté 95 % de monomères d'IgG.

Le mécanisme réactionnel de la conjugaison est le suivant :

$$Ac\text{-}\underset{\underset{O}{\|}}{C}\text{-}H + NH_2\text{-}NH\text{-}VBL \rightarrow \text{-}CH{=}N\text{-}NH\text{-}VBL \bullet$$

Exemple 3:Effet du pH et du solvant sur le couplage

Les IgG non-spécifiques de chèvre ont été oxydées suivant la méthode 1a.

Elles ont été conjuguées par la suite suivant la procédure 2a en utilisant quatre types de conditions

différentes :

a. tampon $PO_4$ pH 5 + dioxanne
b. tampon $PO_4$ pH 6 + dioxanne
c. tampon $PO_4$ pH 7 + dioxanne
d. tampon $PO_4$ pH 6 + DMF.

Aux IgG, à la concentration de 1,8 mg/ml dans le tampon, on ajoute 1:10 en volume d'une solution 5 mM de VBL-NH-$NH_2$ dans le solvant correspondant (DMF ou dioxanne).

Les solutions sont agitées 4 h à 20°C dans le noir, puis dialysées extensivement contre du NaCL 9 ‰.

Le tableau I ci-dessous montre l'effet du pH et du solvant sur le couplage VBL-NH-$NH_2$/IgG par oxydation des sucres.

Tableau I

| Essai | IgG | Oxydation | Couplage | Rapport VBL-NH-$NH_2$/IgG | HPLC % monomères d'IgG |
|---|---|---|---|---|---|
| a | non-spécifique | 4°C 1 h tampon $PO_4$ pH 6 | 4 h 20°C tampon $PO_4$ pH 6 dioxanne | 3,4 | 54 % |
| b | non-spécifique | 4°C 1 h tampon $PO_4$ pH 6 | 4 h 20°C tampon $PO_4$ pH 6 dioxanne | 2,2 | 85 % |
| c | non-spécifique | 4°C 1 h tampon $PO_4$ pH 6 | 4 h 20°C tampon $PO_4$ pH 7 dioxanne | 2 | 85 % |
| d | non-spécifique | 4°C 1 h tampon $PO_4$ pH 6 | 4 h 20°C tampon $PO_4$ pH 6 DMF | 1,5 | 91 % |

Exemple 4 : Estimation du degré d'oxydation des immunoglobulines G

Le degré d'oxydation des IgG a été évalué selon la méthode décrite par R. Radcliffe : à 0,5 mg (0,25 ml) d'IgG oxydée, on ajoute du p-nitrophényl hydrazine (PNH) jusqu'à une concentration finale de 1 mM. Après avoir laissé réagir pendant une heure à température ambiante, le mélange est passé à travers une colonne Sephadex G25 (1,6 x 12 cm). Le pic d'exclusion est collecté, ajusté à pH 8,5 avec NaOH et dosé par spectrométrie à 395 et 280 nm.

Le degré d'oxydation est calculé à l'aide de la formule :

$$\frac{\text{Mole PNH}}{\text{Mole IgG}}$$

Le degré d'oxydation obtenu varie de 7 à 10 quelque soit la méthode d'oxydation utilisée.

Exemple 5 : Digestion enzymatique des conjugués IgG-VBL-NH-$NH_2$ préparés suivant les techniques 2a et 2b de l'exemple 2.

Pour l'étude de la digestion enzymatique, 700 µl de conjugué sont incubés à 37°C pendant 48 h, en présence de cystéine 5 mM, de tampon acétate 40 mM, pH 4,5 et d'enzymes lysosomiales. Au cours du temps des parties aliquotes sont prélevées et les protéines précipitées par addition de 2 volumes de TCA (acide trichloroacétique) 40 %. La digestion est estimée par mesure de la radioactivité soluble dans le surnageant. Après 48 h on mesure 40 % de digestion pour le conjugué de la procédure 2a et 35 % de digestion pour le conjugué 2b.

Ainsi , on a vérifié que les conjugués selon l'invention libéraient bien le principe actif après incubation en présence d'enzymes lysosomiales.

Example 6 : Préparation de conjugués de VBL-NH-$NH_2$ et d'immunoglobuline spécifique $CTMO_1$

Dans les exemples qui suivent, on utilisera plus particulièrement un anticorps dénommé CT-M-O1 qui présente l'ensemble des propriétés énumérées dans la demande de brevet n° 85 10234.

Le protocole expérimental utilisé pour la préparation des Ac monoclonaux dressés contre la membrane de globules graisseux de lait humain est comparable à celui qui a été décrit dans la demande n° 85 10234.

Les immunoglobulines monoclonales ont été oxydées suivant la procédure 1a de l'exemple 1 puis conjuguées suivant la procédure 2a de l'exemple 2, à différents pH.

Le tableau II donne le rapport VBL-NH-$NH_2$/immunoglobuline et le pourcentage de monomères d'immunoglobuline détecté.

| Immunoglobuline | Oxydation | Couplage | Rapport VBL-NH $NH_2$/Ig | HPLC % monomères de Ig |
|---|---|---|---|---|
| $CTMO_1$ | 4°C, 1 h tampon $PO_4^{---}$ pH 6 | 20°C, 4 h tampon $PO_4^{---}$ pH 6 dioxanne | 0,6 | 98 % |
| $CTMO_1$ | 4°C, 1 h tampon $PO_4^{---}$ pH 6 | 20°C, 4 h tampon $PO_4^{---}$ pH 5 dioxanne | 0,9 | 99 % |

Tableau II

Example 7 : Interaction des conjugués avec les cellules MCF-7

On a étudié la réactivité avec la membrane de cellules MCF-7 en culture (cellules d'un carcinome humain de la glande mammaire).

Les cellules $MCF_7$ confluentes ($2.10^6$ cellules/25 $cm^2$) sont incubées à 4°C en présence de différentes concentrations d'anticorps CTMO 1, d'anticorps couplés aux Vincaalcaloïdes : VBL monohydrazide - IgG CTM 01 CTM 01 - Vinca 998 (poids moléculaire) et CTM 01 - Vinca 979. Après 90 minutes d'incubation, les cellules sont lavées une fois avec du milieu de culture et une fois avec du PBS avant d'être réincubées 90 minutes à 4°C en présence de 1 µg/ml d'anticorps CTM 01 marqué à l'Iode 123 (CTM 01-I123). Après avoir été lavées une fois au milieu et trois fois au PBS, les cellules sont récupérées dans du Doc 1 % pH 11.3. La radioactivité associée aux cellules est comptée en compteur $\gamma$ et les protéines sont dosées par la méthode de Lowry.

On obtient des résultats de fixations sur les cellules similaires pour l'anticorps CTM 01 seul, les anticorps couplés CTM 01-VINCA 979 (CTM 01-deacetyl VBL hemisuccinate) et CTM 01-VINCA 998 (CTM 01-VBL monohydrazide).

On obtient des résultats de fixations sur les cellules similaires pour l'anticorps CTM 01 seul, les anticorps couplés CTM 01-VINCA 979 et CTM 01-VINCA 998.

On a donc ainsi vérifié que les conjugués, selon l'invention, conservaient leur affinité d'anti-corps pour les antigènes présents à la surface des cellules MCF 7.

Exemple 8 : Activité des conjugués selon l'invention

Des cultures non confluentes de cellules MCF-7 ont été incubées pendant 24 h à 37°C en présence de différentes concentrations de :

. VBL monohydrazide (PM = 765)
. VBL monohydrazide - IgG CTM 01 (998)
. VBL monohydrazide - IgG chèvre (PM = 974).

A la fin de l'incubation, les cellules ont été lavées (trois fois avec du PBS) et testées pour leur contenu en protéines (méthode de Lowry).

On observe un effet cytotoxique amélioré dans les cellules MCF-7 pour les conjugués hydrazides.

**Revendications**

1. Conjugués actifs incorporant une substance à activité anticancéreuse et un agent de pilotage du type immunoglobuline, la substance à activité cancéreuse et l'agent de pilotage étant liés par un lien convalent du type hydrazide, caractérisée en ce qu'ils sont constitués de dérivés d'hydrazide d'alcaloïde de vinca lié à une immunoglobuline.

2. Conjugués selon l'une des revendications 1 ou 2, caractérisés en ce qu'ils sont constitués d'hydrazide de vinblastine de formule générale (I) :

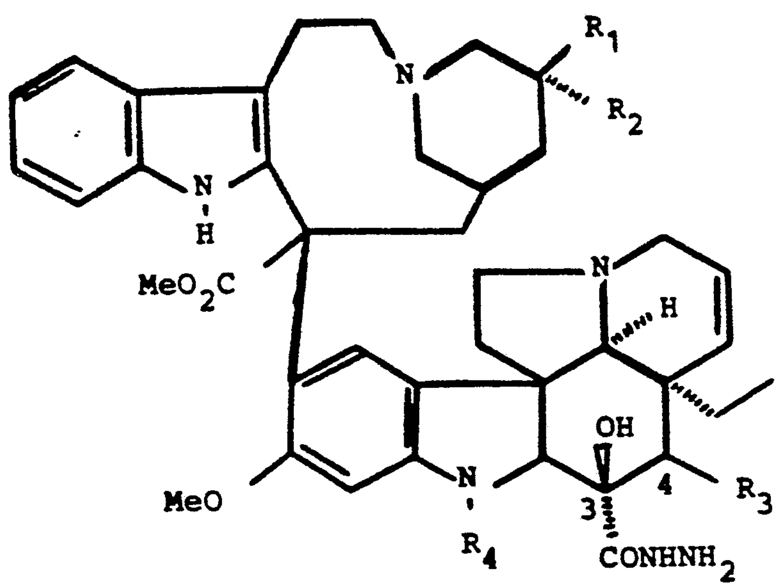

dans laquelle :

. ($R_1$, $R_2$) représentent (-Et, -OH) ou (-H, Et)

. $R_4$ représente -H, -Me ou -CHO,
. $R_3$ représente -OH ou -O- $\underset{\underset{O}{\|}}{C}$ -R'$_3$ ,
. $R'_3$ représente un radical hydrocarboné ayant de 1 à 20 atomes de carbone, le radical hydrocarboné pouvant être non substitué ou substitué par un ou plusieurs radicaux amino, di ($C_1$-$C_3$ alkyl)amino, mono ($C_1$-$C_3$ alkyl)amino, NH ($C_2$-$C_5$)alcanoyl, cyano, COOH, S-($C_1$-$C_3$ alkyl), COO-($C_1$-$C_3$)alkyl ou COO-aryle.
l'hydrazide de vinblastine étant lié à une immunoglobuline.

3. Conjugués selon l'une des revendications précédentes, caractérisés en ce que la liaison entre l'hydrazide et l'immunoglobuline est réalisée au niveau de la région Fc de celle-ci.

4. Conjugués selon l'une des revendications précédentes, caractérisés en ce que l'immunoglobuline est un anticorps monoclonal d'origine animale ou humaine, du type IgG.

5. Conjugués selon l'une des revendications précédentes, caractérisés en ce que l'immunoglobuline est un anticorps monoclonal dressé contre la membrane de globules graisseux de lait humain ou contre la membrane plasmique de cellules de carcinomes humains.

6. Conjugués selon l'une des revendications précédentes, caractérisés en ce que l'immunoglobuline est l'anticorps CTM 01 ou une immunoglobuline IgG de chèvre.

7. Dérivés selon l'une des revendications précédentes, caractérisés en ce que le rapport VBL-NH-$NH_2$/immunoglobuline en nombre de molécules est compris entre 0,5 et 4.

8. Procédé de préparation de conjugués actifs incorporant une substance à activité anticancéreuse et un agent de pilotage du type immunoglobuline, caractérisé en ce qu'on fait réagir dans un solvant organique un dérivé hydrazide de vinblastine comme substance à activité cancéreuse sur une immunoglobuline oxydée, l'hydrazide de vinblastine étant de formule générale I :

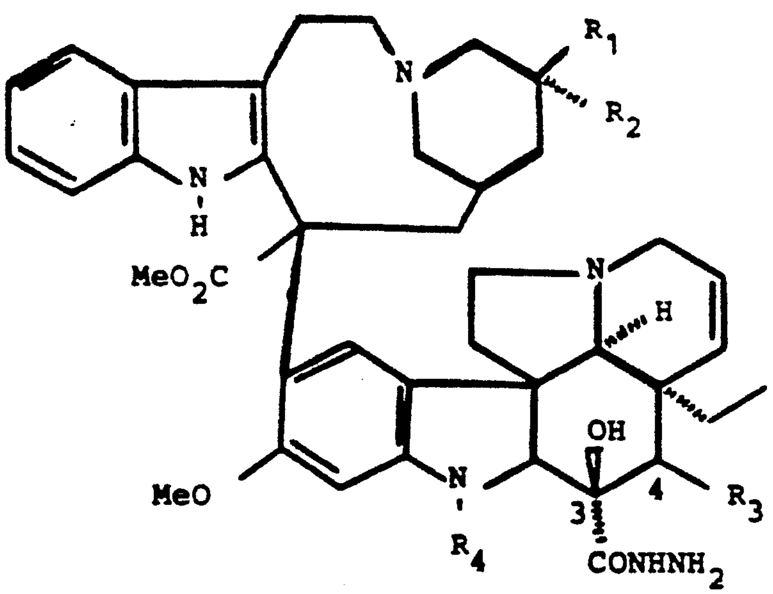

dans laquelle :
. ($R_1$, $R_2$) représente (-Et, -OH) ou (-H, Et)
. $R_4$ représente -H, -Me ou -CHO,
. $R_3$ représente -OH ou -O- $\underset{\underset{O}{\|}}{C}$ -R'$_3$ ,
. $R'_3$ représente un radical hydrocarboné ayant de 1 à 20 atomes de carbone, le radical hydrocarboné pouvant être non substitué ou substitué par un ou plusieurs radicaux amino, di ($C_1$-$C_3$ alkyl)amino, mono ($C_1$-$C_3$ alkyl)amino, NH ($C_2$-$C_5$)alcanoyl, cyano, COOH, S-($C_1$-$C_3$ alkyl), COO-($C_1$-$C_3$)alkyl ou COO-aryle.
l'hydrazide de vinblastine étant lié à une immunoglobuline.

9. Procédé selon la revendication 8, caractérisé en ce qu'on fait réagir le dérivé hydrazide sur la région Fc de l'immunoglobuline oxydée.

10. Procédé selon l'une des revendications 8 ou 9, caractérisé en ce que l'oxydation des immunoglobulines se fait par oxydation des sucres en aldéhydes.

11. Procédé selon l'une des revendications 8 à 10, caractérisé en ce que l'immunoglobuline est du type IgG.

12. Procédé selon l'une des revendications 8 à 11, caractérisé en ce que l'immunoglobuline est l'anticorps CTM 01 ou une immunoglobuline IgG de chèvre.

13. Procédé selon l'une des revendications 8 à 12, caractérisé en ce que le rapport VBL-NH-$NH_2$/immunoglobuline en nombre de molécules est compris entre 0,5 et 4.

14. Procédé selon l'une des revendications 8 à 13, caractérisé en ce que la conjugaison, entre le dérivé hydrazide et l'immunoglobuline, se fait dans un solvant organique à température ambiante dans une solution tampon à pH entre 5 et 7.

15. Procédé selon l'une des revendications 8 à 14, caractérisé en ce que l'oxydation de l'immunoglobuline à lieu dans une solution tamponnée à pH entre 5 et 7, à froid, par adjonction d'une solution d'iodate de sodium.

16. Procédé selon l'une des revendications 14 et 15, caractérisé en ce que les solutions tampons sont des solutions de phosphate de sodium.

17. Procédé selon l'une des revendications 8 à 16, caractérisé en ce que le solvant organique est du dioxanne ou du DMF.

18. Composition pharmaceutique caractérisée en ce qu'elle comprend, à titre de principe actif, un dérivé conforme à l'une des revendications précédentes.

Office européen des brevets

# RAPPORT DE RECHERCHE EUROPEENNE

Numero de la demande

EP 88 40 0220

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 175 617 (CYTOGEN CORP.) * Page 6, lignes 4-11; page 8, ligne 29 - page 11, ligne 12; page 12, ligne 31 - page 13, ligne 23; page 20, lignes 1-7; page 26, lignes 21-29; page 32 - page 35, ligne 23; pages 39-40; tableau I, ligne 26; page 56, lignes 25-31; revendications * | 1,3,4,7 | C 07 D 519/04<br>A 61 K 39/395<br>A 61 K 47/00 |
| Y | | 2,5,8-18 | |
| X | EP-A-0 206 667 (ELI LILLY & CO.) * Page 2, lignes 1-12; page 7, lignes 29-32; pages 14-15; formula IIa; page 18, exemple I; pages 28-33 * | 1,4,7 | |
| Y | | 2,5,8-18 | |
| Y | EP-A-0 208 615 (IRE-CELLTARG S.A.) * En entier * | 5,6 | |
| A | US-A-4 203 898 (ELI LILLY & CO.) | | **DOMAINES TECHNIQUES RECHERCHES (Int. Cl.4)** |
| A | EP-A-0 088 695 (CYTOGEN CORP.) | | A 61 K<br>C 07 D |

Le présent rapport a été établi pour toutes les revendications

| Lieu de la recherche | Date d'achèvement de la recherche | Examinateur |
|---|---|---|
| LA HAYE | 17-06-1988 | SKELLY J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)